# EUROPEAN PATENT SPECIFICATION

(11) **EP 4 581 566 B1**
(45) Date of publication and mention of the grant of the patent: **04.03.2026**
(21) Application number: 23754230.3
(22) Date of filing: 04.08.2023
(51) Int. Cl.: G06T 7/00, G06T 11/00

(54) **OPTIMIZING CT IMAGE FORMATION IN SIMULATED X-RAYS**
OPTIMIERUNG DER CT-BILDERZEUGUNG IN SIMULIERTEN RÖNTGENSTRAHLEN
OPTIMISATION DE FORMATION D'IMAGE TDM EN RAYONS X SIMULÉS

(30) Priority: 01.09.2022 US 202263402981 P; 09.03.2023 US 202363450987 P
(43) Date of publication of application: 09.07.2025
(73) Proprietor: Koninklijke Philips N.V., 5656 AG Eindhoven (NL)
(72) Inventor: WUELKER, Christian, 5656 AG Eindhoven (NL); SEVENSTER, Merlijn, 5656 AG Eindhoven (NL); LAMB, Hildo, 5656 AG Eindhoven (NL); PAALVAST, Olivier, 5656 AG Eindhoven (NL)
(74) Representative: Philips Intellectual Property & Standards
(86) International application number: PCT/EP2023/071703
(87) International publication number: WO 2024/046711

(56) References cited:
- US-A1- 2022 180 447
- YUXING TANG ET AL: "TUNA-Net: Task-oriented UNsupervised Adversarial Network for Disease Recognition in Cross-Domain Chest X-rays", ARXIV.ORG, CORNELL UNIVERSITY LIBRARY, 201 OLIN LIBRARY CORNELL UNIVERSITY ITHACA, NY 14853, 21 August 2019 (2019-08-21), XP081466922
- YUE ZHANG ET AL: "Task Driven Generative Modeling for Unsupervised Domain Adaptation: Application to X-ray Image Segmentation", ARXIV.ORG, CORNELL UNIVERSITY LIBRARY, 201 OLIN LIBRARY CORNELL UNIVERSITY ITHACA, NY 14853, 12 June 2018 (2018-06-12), XP081763687, DOI: 10.1007/978-3-030-00934-2_67

## Description

### FIELD OF THE INVENTION

The present invention generally relates to systems and methods for simulating conventional X-ray images from computed tomography (CT) data. In particular, the present invention relates to optimizing simulated X-ray images based on disease or condition classification using tunable parameters in models of an image formation chain.

### BACKGROUND OF THE INVENTION

CT imaging provides advantages over conventional planar X-ray (CXR) imaging. Accordingly, CT imaging has replaced X-ray imaging in various clinical settings and is increasingly adopted in additional clinical settings in place of such X-ray imaging.

This is particularly true in the case of low-dose and ultra-low-dose CT imaging (ULDCT), which now aims at replacing CXR in additional settings, such as routine chest imaging in outpatient settings.

One main advantage of CT imaging over CXR is that CT provides additional information and, in particular, three-dimensional spatial information. Also, CXR has a relatively low sensitivity and high false negative rate in many clinical scenarios. CT imaging, because of the additional information associated with it, is also more amenable to various image processing and artificial intelligence (AI) based diagnosis techniques.

On the other hand, CXR has a higher spatial resolution and suffers less from noise than conventional CT imaging and, in particular, ULDCT imaging.

One reason that CT imaging has not been more widely adopted in routine clinical settings is that reading time of CT imaging is substantially higher than CXR. This is partially because radiologists are more familiar with CXR images and are therefore more comfortable reading and basing diagnoses on such conventional planar X-ray images.

However, to the extent that radiologists continue to rely on CXR images, they are forgoing the advantages of CT imaging, both in terms of additional information and analytical capability.

Accordingly, it is known to create digitally reconstructed radiograph (DRR) images in order to incorporate some of the advantages associated with CT images into a synthetic CXR image. Such DDR images may be constructed by digitally tracing X-Rays through a 3D CT volume. In a clinical setting, such reconstructions may be used by radiologists in various settings where a CXR image would traditionally be used.

Classification models for a disease, condition, disorder, etc. (hereinafter collectively referred to as "disease classification models") are known in the context of CXR images. However, such models cannot be applied to CT images themselves, and as such, where image processing is applied to CT data prior to generating the DDR, information related to disease classification cannot be leveraged to optimize reconstruction.

US patent application US2022/180447A1 discloses a 3D-to-2D mapping of a volumetric grid image from a CBCT, MM, or CT scan, using a ray tracing algorithm to obtain a virtual 2D image.

### OBJECT OF THE INVENTION

There is a need for a CT imaging system and method and, in particular, an ULDCT imaging system and method that can present data to radiologists in a form more easily interpreted and more likely to be adopted. There is a further need for such a system in which disease classification can be leveraged to optimize image reconstruction.

Systems and methods for transforming three-dimensional CT data into two dimensional images according to the independent claims are provided. Such a method and system includes retrieving projection data acquired by scanning a subject from a plurality of angles about a central axis, for example.

In some embodiments, generating the two-dimensional image is performed by a neural network.

In some embodiments, reprocessing the projection data or the three-dimensional image is performed by a neural network

In some embodiments, the three-dimensional image is denoised using a first value for the at least one parameter. In some such embodiments, the updated three-dimensional image is denoised using a second value for the at least one parameter. The second value is based on the disease or the uncertainty in the identification of the disease.

In some embodiments, applying, an artificial intelligence (AI) based super-resolution is applied to the three-dimensional image using a first value for the at least one parameter, such that the three-dimensional image results in a high resolution image. In some such embodiments, the AI based super-resolution is applied to the updated three-dimensional image using a second value for the at least one parameter, such that the updated three-dimensional image results in another high resolution image. The second value is based on the disease or the uncertainty in the identification of the disease.

In some embodiments, the three-dimensional image and/or the updated three-dimensional image is denoised by a trained convolutional neural network (CNN).
In some embodiments, the two-dimensional image is generated by a DRR network using a first value for the at least one parameter. In some such embodiments, the updated two-dimensional image is generated by using the DRR network using a second value for the at least one parameter. The second value is based on the disease or the uncertainty in the identification of the disease.

In some embodiments, at least one physical element is identified in the three-dimensional image and/or the updated three-dimensional image. The at least one physical element is removed or masked out prior to generating an output image. In some such embodiments, the at least one physical element is a plurality of ribs or a heart. In some such embodiments, the at least one physical element is removed or masked out based on the disease or the uncertainty in the identification of the disease in the two-dimensional image.

In some embodiments, the disease is identified by a use of a trained disease classification model.

In some embodiments, the generating of the two-dimensional image and the reprocessing of the projection data or the three-dimensional image are performed by a neural network.

In some embodiments, the three-dimensional image is denoised using a first value for the at least one parameter. In some such embodiments, the updated three-dimensional image is denoised using a second value for the at least one parameter. The second value is based on the disease or the uncertainty in the identification of the disease.

In some embodiments, the three-dimensional image and/or the updated the three-dimensional image is denoised by a trained convolutional neural network (CNN).

In some embodiments, the two-dimensional image is generated by a DRR network using a first value for the at least one parameter. In some such embodiments, the updated two-dimensional image is generated by the DRR network using a second value for the at least one parameter. The second value based on the disease or the uncertainty in the identification of the disease.

In some embodiments, an AI based super-resolution using a first value for the at least one parameter is applied to the three-dimensional image such that the three-dimensional image results in a high resolution image. In some such embodiments, the AI based super-resolution using a second value for the at least one parameter is applied to the updated three-dimensional image such that the updated three-dimensional image results in another high resolution image. The second value is based on the disease or the uncertainty in the identification of the disease.

### BRIEF DESCRIPTION OF THE DRAWINGS

Figure 1 is a schematic diagram of a system according to one embodiment of the present invention.
Figure 2 illustrates an exemplary imaging device according to one embodiment of the present invention.
Figure 3 illustrates an image formation chain for use in a method of one embodiment of the present invention.
Figure 4 illustrates a schematic overview of a model structure for implementing a method of one embodiment of the present invention.
Figure 5 is a flow chart illustrating a method in accordance with one embodiment of the present invention.
Figure 6 schematically shows a ray tracing process applied to a three-dimensional image usable in the context of one embodiment of the present invention.
Figure 7 is a flow chart illustrating a method for training an image formation chain in accordance with one embodiment of the present invention.

### DETAILED DESCRIPTION OF THE PREFERRED EMBODIMENTS

The description of illustrative embodiments according to principles of the present invention is intended to be read in connection with the accompanying drawings, which are to be considered part of the entire written description. In the description of embodiments of the invention disclosed herein, any reference to direction or orientation is merely intended for convenience of description and is not intended in any way to limit the scope of the present invention. Relative terms such as "lower," "upper," "horizontal," "vertical," "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise. Moreover, the features and benefits of the invention are illustrated by reference to the exemplified embodiments. Accordingly, the invention expressly should not be limited to such exemplary embodiments illustrating some possible non-limiting combination of features that may exist alone or in other combinations of features; the scope of the invention being defined by the claims appended hereto.

This invention describes the best mode or modes of practicing the invention as presently contemplated. This description is not intended to be understood in a limiting sense, but provides an example of the invention presented solely for illustrative purposes by reference to the accompanying drawings to advise one of ordinary skill in the art of the advantages and construction of the invention. In the various views of the drawings, like reference characters designate like or similar parts.

It is important to note that the embodiments disclosed are only examples of the many advantageous uses of the innovative teachings herein. In general, statements made in the specification of the present application do not necessarily limit any of the various claimed embodiments. Moreover, some statements may apply to some inventive features but not to others. In general, unless otherwise indicated, singular elements may be in plural and vice versa with no loss of generality.

Both computed tomography (CT) and conventional planar X-ray (CXR) are used in medical imaging. However, CT imaging and, in particular, ultra-low-dose CT imaging (ULDCT) aim at replacing CXR in many clinical settings such as, for example, chest imaging in routine outpatient settings.

Some of the main advantages of ULDCT imaging are immediately apparent. CT imaging, including ULDCT, provides three-dimensional spatial information, which allows for sophisticated analytical techniques. Further, ULDCT avoids the relatively low sensitivity and high false negative rates associated with CXR in many clinical scenarios.

However, ULDCT has a slower read time than CXR, and radiologists are less familiar and less comfortable with ULDCT. As such, radiologists prefer to be presented with and make diagnoses based on more familiar CXR images. The embodiments of the present invention therefore provide a workflow for generating artificial CXR images, or images stylized to have the appearance of CXR images, from ULDCT data. Such embodiments may be implemented or enhanced using artificial intelligence (AI) techniques, including the use of learning algorithms in the form of neural networks, such as convolutional neural networks (CNN).

Accordingly, methods are provided for transforming three-dimensional CT data into two-dimensional images. In this way, CXR style images may be generated from ULDCT data and presented to radiologists. Such presentation may follow the implementation of analytical techniques to the underlying ULDCT data in either raw or three-dimensional image formats, and may be presented to radiologists either as a proxy for a CXR image or in the context of a corresponding ULDCT based image interface.

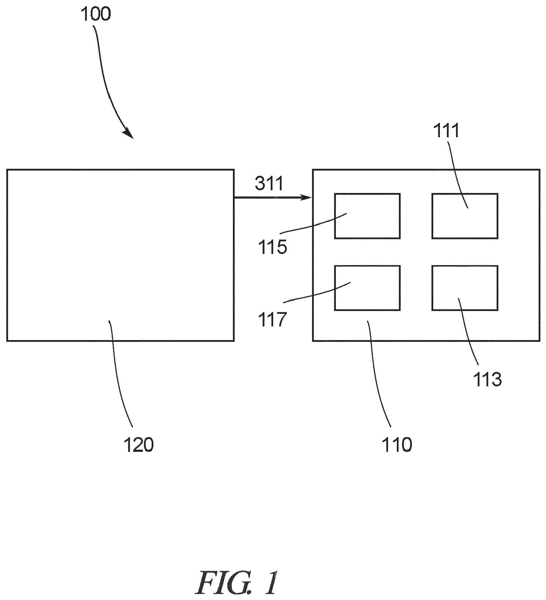

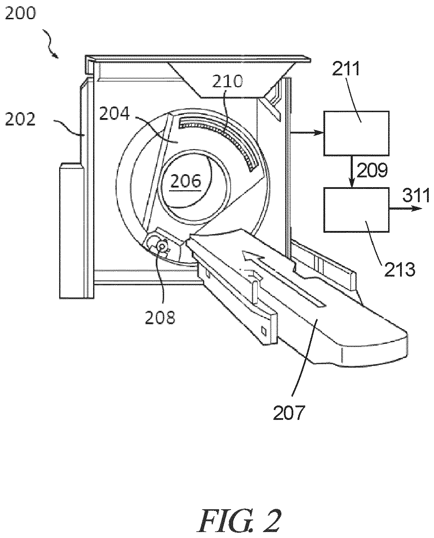

Accordingly, ULDCT imaging data may be generated as three-dimensional CT imaging data using a system such as that illustrated in Figure 1 and by way of an imaging device such as that illustrated in Figure 2. The retrieved data may then be processed using the processing device of the system of Figure 1.

Once the ULDCT imaging data is generated, the CT imaging data may be processed as a three-dimensional image by passing it through an image formation chain. Such an image formation chain may include a denoising process, a super-resolution process, and a DRR simulation for converting the three-dimensional image to a two-dimensional image. The image formation chain may include a plethora of parameters which may be tuned and which affect the resulting DRR image. It is unfeasible from a practical perspective to iterate over an infinite number of possible images in order to identify which image is optimal for a given case. Additionally, it is not always clear what makes an image best in a certain situation.

In the setting of evaluating medical images, this is especially relevant as different radiologists might have different opinions on what aspects of an image they find relevant. Further, such expertise is not always readily available. The issue of "expensive" medical expertise is well-known in the use of AI in disease classification.

Accordingly, one embodiment of the present invention uses an AI model that links the image formation chain to a pre-trained disease classification model. It is then possible to evaluate and optimize the effect of modifying tunable parameters in the image formation chain. Accordingly, the pre-trained disease classification model may evaluate a first attempt at transforming a three-dimensional CT image to a two-dimensional image and classify the image accordingly. The method may then modify tunable parameters in the image formation chain and reprocess the projection data or the 3-D images using the tuned parameters associated with an identified disease.

Figure 1 is a schematic diagram of a system 100 according to one embodiment of the present invention. As shown, the system 100 typically includes a processing device 110 and an imaging device 120.

The processing device 110 may apply processing routines to images or measured data, such as projection data, received from the image device 120. The processing device 110 may include a memory 113 and processor circuitry 111. The memory 113 may store a plurality of instructions. The processor circuitry 111 may couple to the memory 113 and may be configured to execute the instructions. The instructions stored in the memory 113 may comprise processing routines, as well as data associated with processing routines, such as machine learning algorithms, and various filters for processing images.

The processing device 110 may further include an input 115 and an output 117. The input 115 may receive information, such as 3-D images or projection data from the imaging device 120. The output 117 may output information, such as filtered images or converted two-dimensional images, to a user or a user interface device. The output may include a monitor or display.

In some embodiments, the processing device 110 may relate to the imaging device 120 directly. In alternate embodiments, the processing device 110 may be distinct from the imaging device 120, such that the processing device 110 receives images or measured data for processing by way of a network or other interface at the input 115.

In some embodiments, the imaging device 120 may include an image data processing device, i.e., a spectral or conventional CT scanning unit for generating projection data when scanning a subject (e.g., a patient). In some embodiments, the imaging device 120 may be a conventional CT scanning unit configured for generating helical scans.

Figure 2 illustrates an exemplary imaging device 200 according to one embodiment of the present invention. It will be understood that while a CT imaging device 200 is shown, and the following discussion is generally in the context of CT images, similar methods may be applied in the context of other imaging devices, and images to which these methods may be applied may be acquired in a wide variety of ways.

In an imaging device 200 in accordance with embodiments of the present invention, the CT scanning unit may be adapted for performing one or multiple axial scans and/or a helical scan of a subject in order to generate the CT projection data. In an imaging device 200 in accordance with embodiments of the present invention, the CT scanning unit may comprise an energy-resolving photon counting and/or spectral dual-layer image detector. Spectral content may be acquired using other detector setups as well. The CT scanning unit may include a radiation source that emits radiation for traversing the subject when acquiring the projection data.

In the example shown in FIG. 2, the CT scanning unit 200, e.g. the CT scanner, may include a stationary gantry 202 and a rotating gantry 204, which may be rotatably supported by the stationary gantry 202. The rotating gantry 204 may rotate about a longitudinal axis around an examination region 206 for the subject (patient) when acquiring the projection data. The CT scanning unit 200 may include a table or support 207 to support the patient in the examination region 206 and configured to pass the patient through the examination region during the imaging process.

The CT scanning unit 200 may include a radiation source 208, such as an X-ray tube, which may be supported by and configured to rotate with the rotating gantry 204. The radiation source 208 may include an anode and a cathode. A source voltage applied across the anode and the cathode may accelerate electrons from the cathode to the anode. The electron flow may provide a current flow from the cathode to the anode, such as to produce radiation for traversing the examination region 206.

The CT scanning unit 200 may comprise a detector 210. The detector 210 may subtend an angular arc opposite the examination region 206 relative to the radiation source 208. The detector 210 may include a one- or two-dimensional array of pixels, such as direct conversion detector pixels, for example. The detector 210 may be adapted for detecting radiation traversing the examination region 206 and for generating a signal indicative of an energy thereof.

The CT scanning unit 200 may include generators 211 and 213. The generator 211 may generate projection data 209 based on the signal from the detector 210. The generator 213 may receive the projection data 209 and, in some embodiments, generate three-dimensional images 311 of the subject based on the projection data 209. In some embodiments, the projection data 209 may be provided to the input 115 of the processing device 110, while in other embodiments the 3-D images 311 are provided to the input of the processing device.

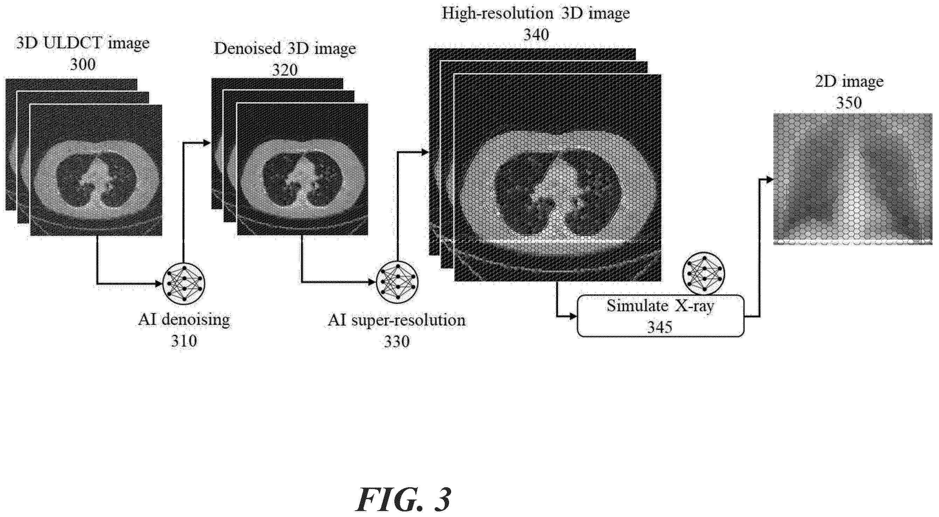

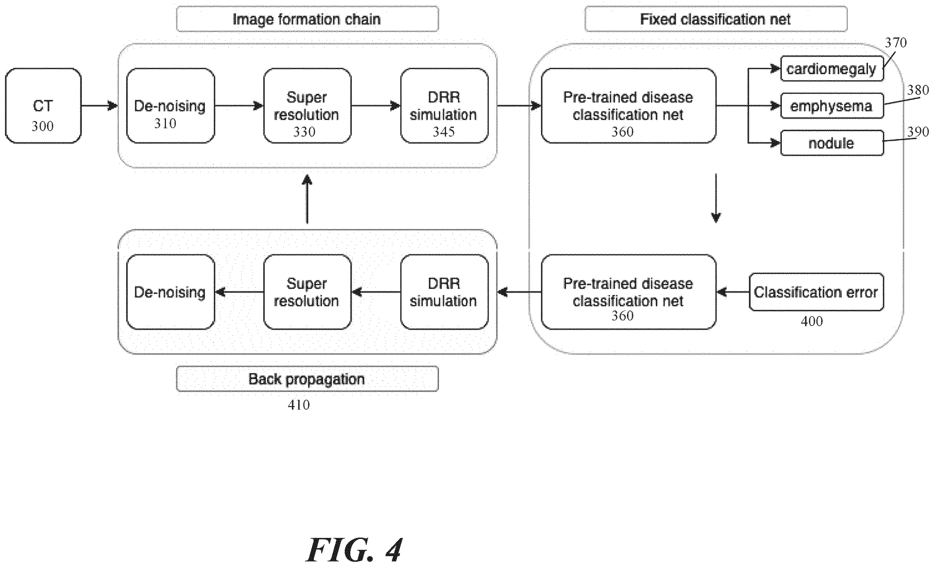

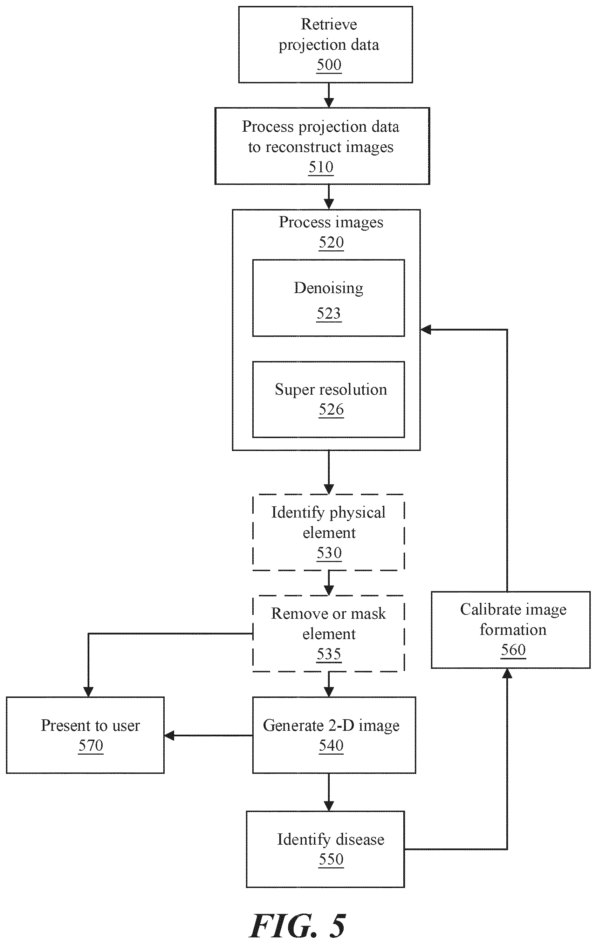

Figure 3 illustrates an image formation chain for use in a method/apparatus of one embodiment of the present invention. Figure 4 illustrates a schematic overview of a model structure for implementing a method of one embodiment of the present invention. Figure 5 is a flow chart illustrating a method in accordance with one embodiment the present invention.

As shown, according to the method of one embodiment, CT data is processed into images and typically includes retrieving (500) projection data acquired from a plurality of angles about a central axis.

Accordingly, in the context of the imaging device 200 of FIG. 2, the subject may be a patient on the support 207, and the central axis may be an axis passing through the examination region. Where the projection data is acquired from the imaging device 200, the rotating gantry 204 may then rotate about the central axis of the subject, thereby acquiring the projection data from various angles.

Once acquired, the projection data is processed to reconstruct (510) 3-D images 300, which are then processed (520).

It is understood that while the reconstruction (510) and the processing (520) are indicated as distinct processes, the reconstruction itself may be the actual processing of the projection data into 3-D images. Similarly, the reconstruction may be part of such processing. Such reconstruction (510) may be by using standard reconstruction techniques, such as by way of filtered back projection.

As shown in FIG. 3 and FIG. 5, the processing (520) may include denoising 310 (523) which may be, for example, by way of a neural network or other algorithm, such as an AI based learning algorithm. In the example shown, the denoising 310 (523) is by way of a convolutional neural network (CNN) previously trained on appropriate images. Such denoising processes 310 (523) may be utilized, for example, where the CT imaging is noisy, as in the case of ULDCT images. The denoising process (523) may then result in a denoised or partially denoised 3-D image 320.

The denoising process 310 (523) may be a process that incorporates features that allow it to generalize well to different contrasts, anatomies, reconstruction filters, and noise levels. Such denoising process 310 may compensate for the high noise levels inherent in ULDCT images. The denoising process 310 (523) includes denoising the imaging data using a denoising algorithm with at least one tunable parameter. During an initial processing (520), the tunable parameter is typically set to a first value. The first value may be based on a generic, or non-diseased image, or it may be based on an expected disease associated with the CT imaging.

In the example shown in the figures, the processing of the three-dimensional images (520) may further include the implementation of a super-resolution process 330 (526). As in the case of the denoising process 310, the super-resolution process 330 (526) may be by way of a neural network or other algorithm, such as an AI based learning algorithm, such as CNN, for example. In some embodiments, the super-resolution process 330 may include deblurring of the image. The super-resolution process 330 results in a higher resolution three-dimensional image 340.

The super-resolution process 330 typically interpolates the image to smaller voxel sizes, while maintaining perceived image sharpness or improving perceived image sharpness. Alternatively, the super-resolution process 330 may operate on a fixed voxel size and perform a deconvolution-like operation in order to restore a higher resolution form a blurred image. AI based super-resolution processes 330 may be trained on either real CT images, including ULDCT images, or more generic image material, such as natural high-resolution photos. The super-resolution process 330 (526) may include performing an AI based super-resolution process using a super-resolution algorithm with at least one tunable parameter. During an initial processing (520), the tunable parameter is typically set to a first value. The first value may be based on a generic, or non-diseased image, or it may be based on an expected disease associated with the CT imaging.

In the embodiment shown in the figures, both denoising processes 310 (523) and super-resolution processes 330 (526) are applied in sequence. However, it will be understood that both processes may be incorporated into a single neural network, such as a CNN. Further, while both processes 310, 330 are shown as applied to the 3-D image 300, in some embodiments, the processes may be applied directly to the projection data prior to reconstruction (510). Further, in some embodiments, one or both processes 310, 330 may be applied on two-dimensional planes in the three-dimensional CT imaging data set perpendicular to the projection direction to be used to generate the two-dimensional image discussed below.

In some embodiments, processing may further comprise identifying (530) at least one physical element in the 3-D image. Once identified (530), the physical element may be removed or masked out (535) of the 3-D image. By removing or masking out (535) an element prior to the generation of a 2-D image, such a physical element may be removed from a simulated X-ray to be generated from the projection data.

The identified physical element (530) may be an anatomical element, such as one or more ribs or a heart. By removing such an anatomical element from a simulated X-ray, other anatomical elements of interest to a radiologist viewing the images may be more easily visible, and the simulated X-ray may show a cutaway of a patient's chest cavity without interfering ribs, for example.

Alternatively, the identified physical element (530) may be a table or support 207 or an implant. CT imaging data is typically acquired from a patient lying on a table or support 207, as in the imaging device 200 discussed above. In contrast, conventional planar X-rays are often acquired from standing patients. Accordingly, by removing a support 207, a simulated X-ray may appear more natural to a radiologist viewing the images. Similarly, removing an implant may provide a better view of a patient's anatomy.

In some embodiments, rather than removing the identified physical element (530), the physical element may instead be weighted. Similarly, different sections of the three-dimensional image 300 may be weighted differently. The removal, masking out, or weighting of a physical element identified (530) may be based on an algorithm having a tunable parameter. As such, and as discussed in the context of denoising (523) and super-resolution (526) processing, the tunable parameter may be provided with a first value prior to initial processing.

Following the processing of the 3-D images (520), the method proceeds to generate a two-dimensional image 350 (540) based on the three-dimensional image. While this may be performed in a number of ways, in some embodiments such generation of a two-dimensional image 350 is by tracing X-rays from a simulated radiation source outside of a subject of the three-dimensional image. Such an X-ray tracing process may be, for example, by implementing a Siddons-Jacobs ray-tracing algorithm.

Once a two-dimensional image 350 has been generated, the method proceeds to identify a disease represented in the two-dimensional image (550). Such an identification may be by use of a pre-trained disease classification network 360. Such a network may be configured to recognize a specific set of diseases relevant to the particular anatomy appearing in the two-dimensional image 350. For example, chest imaging may be processed with a disease classification network 360 trained to identify cardiomegaly 370, emphysema 380, and individual nodules 380. Accordingly, the disease classification model may be previously trained based on potential diseases. In addition to identifying a specific disease, the network 360 may be designed to output additional parameters of the identification, such as a degree of uncertainty in the results. Accordingly, in embodiments where portions of the three-dimensional image are masked out before generating the updated two-dimensional image 540, basing parameters on such uncertainty in the results may make sense, as a nodule may be identified but partially hidden.

Upon identifying a disease represented in the two-dimensional image 350, the method recalibrates (560) the image formation chain by either 1) reconstructing the projection data as a 3-D image or 2) processing the original reconstructed 3-D image in the image domain, without the need for reconstruction in a projection domain. This results in an updated 3-D image. Such recalibrating is based on the identified disease (550) and/or an output of the pre-trained disease classification network 360. Such recalibration includes modifying some part of the processing of the image (520) such that at least one parameter of the reprocessing is based on the disease identified (550) and/or the output of the network 360. For example, in some embodiments, in addition to the actual identified disease, the recalibration may consider a reported uncertainty of the disease classification network.

As noted above, the processing of the images (520) may include denoising (523) and/or a super-resolution process (526). As noted above, the denoising (523) may utilize a denoising algorithm having a tunable parameter. The recalibration (560) of the image formation chain may then include providing a second value for the tunable parameter of the denoising algorithm (523), different than the first value. The second value may then be based on the identified disease (550). Similarly, the super-resolution process (523) may utilize a super-resolution algorithm having a tunable parameter. The recalibration (560) of the image formation chain may then include providing a second value for the tunable parameter of the super-resolution algorithm (526) different than the first value. As in the case of the denoising algorithm, the second value may be based on the identified disease (550).

Similarly, in embodiments in which physical elements are identified (530) and removed, masked, or weighted (535), the calibration of the image formation chain (560) may set a tunable parameter of an algorithm used to control the identification, removal, masking, or weighting. As such, while the tunable parameter may initially be provided with a first value, the calibration (560) may set it to a second value different than the first. As such, the removing or masking out of the at least one physical element may be based on the identification (550) of the disease represented in the two-dimensional image.

Once the 2-D image, as calibrated via the image formation chain and based on the at least one updated parameter, is generated (540), the method proceeds to present the updated 2-D image to a user (570).

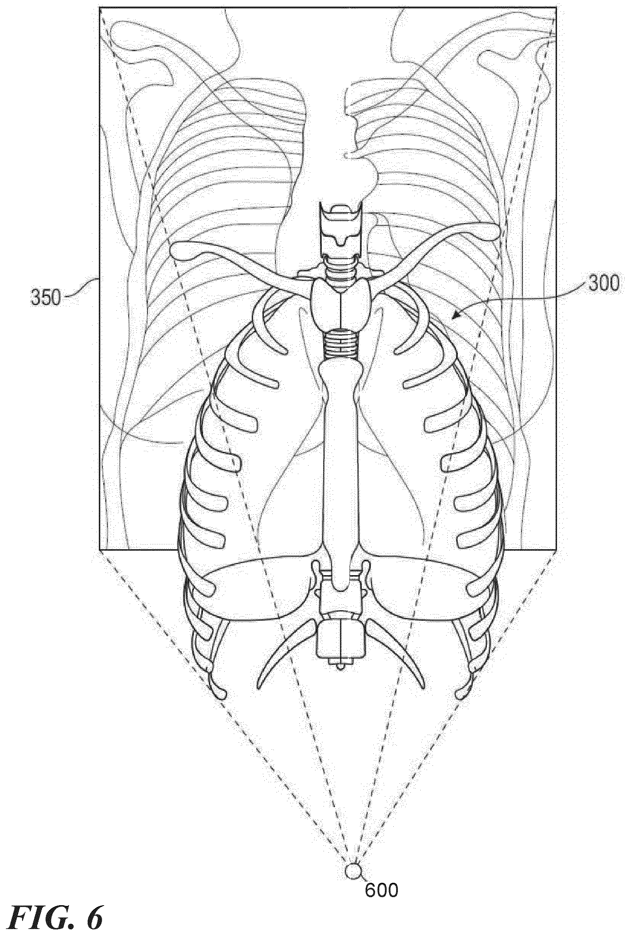

Figure 6 illustrates an implementation of a ray tracing process, applied to a three-dimensional image 300 (540) in order to generate a two-dimensional image 350. The ray tracing process may then proceed by simulating the process of an X-ray 345 by propagating incident X-ray photons from a simulated radiation source 600 through the reconstructed three-dimensional image 300. The generation of the two-dimensional image 350 may be by way of a neural network, such as a CNN, and in such cases, the CNN may incorporate one or more of denoising and super-resolution processes discussed herein. Such a neural network may be a generative adversarial network (GAN). In such an embodiment, many or all of the steps described herein may be incorporated into a single network, such that CT volume data is provided to the network, and simulated CXR projections are output.

In some embodiments, the generation of the two-dimensional image (540) may be based on an algorithm, such as an algorithm utilizing a CNN, having a tunable parameter. Such a CNN may be a network configured to generate a digitally reconstructed radiograph (DRR). As in the case of the tunable parameters of the denoising algorithm (523) and the super-resolution algorithm (526), such a tunable parameter may be provided with a first value during the initial generation of the two-dimensional image (540). In such an embodiment, after identifying a disease (550) and calibrating the image formation chain (560) and during reprocessing of the image, the method may generate an updated two-dimensional image based on a second value for the tunable parameter based on the identification of the disease represented in the two-dimensional image.

In some embodiments, the projection angle or orientation of the ray tracing process (540) may be adjusted in order to improve the resulting two-dimensional image 350. Similarly, weighting of physical elements in the three-dimensional image 300 may be adjusted in order to improve the resulting two-dimensional image 350. Such characteristics may be controllable by at least one tunable parameter that can be set by the calibration process (560).

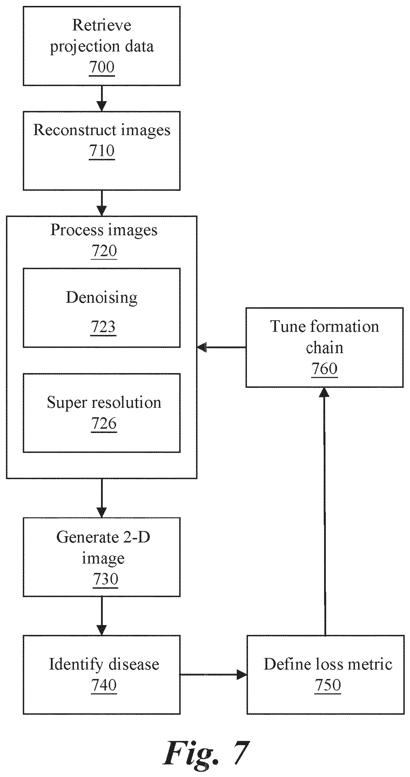

Figure 7 is a flow chart illustrating a method for training an image formation chain in accordance with the present invention. The method begins by retrieving projection data acquired from a plurality of angles about a central axis (700). Accordingly, the acquisition of such training data is similar to that discussed above with respect to FIG. 5 (500).

The training data further includes an indicator of a disease status associated with it. Such an indicator of a disease status may indicate a particular disease, such as cardiomegaly 370, emphysema 380, and the presence of individual nodules 380. Alternatively, the indicator of a disease status may indicate that the training data corresponds to healthy anatomy.

The method then proceeds to reconstruct (710) and process the reconstructed 3-D images (720). The processing of the images (720) is similar to that discussed above with respect to FIG. 5, and proceeds along a similar image formation chain. As such, the processing of the images (720) includes denoising (723) and a super-resolution process (726). Such processing is followed by generating a two-dimensional image (730) based on the training image data.

As discussed above with respect to FIG. 5, the processes incorporated into the image formation chain, including the denoising process (723), the super-resolution process (726), and the generation of the two-dimensional image (730) may each incorporate tunable parameters, which may initially be provided with a default or first value. The method described herein for training the image formation chain may be used to derive disease specific second values for those tunable parameters in order to enhance the output of the image formation chain following the identification of a disease.

Following the generation of the two-dimensional image (730), a previously trained disease classification network is applied (740) to the two-dimensional image output, and is used to generate a disease classification prediction associated with the training image data. A loss metric is then defined (750) based on an output of the previously trained disease classification network and the indicator of a disease status associated with the training image data.

The loss metric may correspond to a classification error 400 which may then be provided to the pre-trained disease classification net. Such a classification error may then be back-propagated 410 (FIG. 4) through the image formation chain in order to identify and refine values for the tunable parameters of the image formation chain. Accordingly, the loss metric (750) is then used to tune (760) at least one parameter of the processing of the training images to minimize the loss metric. As such, because the loss metric is defined (750) on the basis of a disease status indicator, the value for one or more of the tunable parameters identified by the training method are optimized for a specified disease status.

The training process may be repeated multiple times on a large number of training images for a variety of specified disease statuses. Further, the tunable parameters may be a single tunable parameter in one of the denoising process 310, the super-resolution process 330, or the DRR simulation process 345, or it may be, for example, a first tunable parameter in the denoising process 310 and a second tunable parameter in the super-resolution status, each of which are to be tuned by the training method to discover ideal values.

In some embodiments, the at least one parameter impacts suppression of anatomy in an output image generated by processing three-dimensional images using the tuned value for the at least one parameter. Accordingly, the indicator of a disease status may indicate the presence of an identified disease, and the loss metric may optimize the visibility of an indicator of the identified disease. For example, the loss metric may optimize a tunable parameter to increase the visibility of a nodule.

In some such embodiments, the loss metric optimizes the visibility of nodules otherwise obscured by adjacent anatomy. As such, the tuned parameter may result in the masking of blocking anatomy, such as a rib cage, for example.

For example, in the context of chest imaging, identification of lung nodules is notoriously difficult in chest X-rays due to resolution of the film, the size of the nodules, and the potential for nodules to be obscured by adjacent anatomy. In this embodiment, the image formation chain is then optimized towards suppressing obscuring adjacent anatomy in the DRR image simulation and enhancing nodule detail and granularity through the super-resolution and denoising modules respectively.

The individual models within the image formation chain can typically accept the output of the other individual models.

The pre-trained disease classification network expects an image (or in this case DRR) from the domain of the images it has been trained on. Concretely, this means supplying DRR images from chest CT scans if the network has been trained on chest X-rays, etc. The added value of combining these features is the creation of a model which is optimized for a real-world application, such as disease classification, which can simultaneously generate and optimize 2-D representations from 3-D images.

In some embodiments, the image formation chain can be expanded in both directions. In one embodiment, the image formation chain may include a disease classification model based on the underlying three-dimensional images. This could supplement the two-dimensional pre-trained classification model by incorporating contextual and spatial information into the classification for a given case. Similarly, the image formation chain may be expanded to include the reconstruction of CT images, such that learnable parameters may be utilized in the reconstruction process. For example, the visualization of individual CT slides may be improved for specific disease classes.

The various embodiments of the present invention may be implemented on a computer as a computer implemented method, or in dedicated hardware, or in a combination of both. Executable code for a method according to one embodiment of the present invention may be stored on a computer program product. Examples of computer program products include memory devices, optical storage devices, integrated circuits, servers, online software, etc. Preferably, the computer program product may include non-transitory program code stored on a computer readable medium for performing a method according to the present invention when the program product is executed on a computer. In one embodiment, the computer program may include computer program code adapted to perform the method according to various embodiments of the present invention when the computer program is run on a computer. The computer program may be embodied on a computer readable medium.

While the present invention has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the invention.

All examples and conditional language recited herein are intended for pedagogical purposes to aid the reader in understanding the principles of the invention and the concepts contributed by the inventor to furthering the art, and are to be construed as being without limitation to such specifically recited examples and conditions. Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

## Claims

1. A computer-implemented method for processing medical computed tomography (CT) data, comprising:
retrieving projection data acquired by scanning a subject;
processing the projection data to reconstruct a three-dimensional image;
generating a two-dimensional image based on the three-dimensional image;
identifying a disease or an uncertainty in an identification of the disease in the two-dimensional image;
reprocessing the projection data or the three-dimensional image into an updated three-dimensional image, wherein at least one parameter of the reprocessing is based on the disease or the uncertainty in the identification of the disease; and
generating an updated two-dimensional image based on the updated three-dimensional image.

2. The method according to claim 1, wherein generating the two-dimensional image is performed by a neural network.

3. The method according to claim 1, wherein reprocessing the projection data or the three-dimensional image is performed by a neural network.

4. The method according to claim 1, further comprising denoising the three-dimensional image using a first value for the at least one parameter.

5. The method according to claim 4, further comprising denoising the updated three-dimensional image using a second value for the at least one parameter, the second value based on the disease or the uncertainty in the identification of the disease.

6. The method according to claim 1, further comprising applying, to the three-dimensional image, an artificial intelligence (AI) based super-resolution using a first value for the at least one parameter such that the three-dimensional image results in a high resolution image.

7. The method according to claim 6, further comprising applying, to the updated three-dimensional image, the AI based super-resolution using a second value for the at least one parameter such that the updated three-dimensional image results in another high resolution image, wherein the second value is based on the disease or the uncertainty in the identification of the disease.

8. The method according to claim 1, further comprising denoising the three-dimensional image and/or the updated the three-dimensional image by a trained convolutional neural network (CNN).

9. The method according to claim 1, wherein generating the two-dimensional image comprises using a digitally reconstructed radiograph (DRR) network using a first value for the at least one parameter.

10. The method according to claim 9, wherein generating the updated two-dimensional image comprises using the DRR network using a second value for the at least one parameter, the second value based on the disease or the uncertainty in the identification of the disease.

11. The method according to claim 1, further comprising identifying at least one physical element in the three-dimensional image and/or the updated three-dimensional image, and removing or masking out the at least one physical element prior to generating an output image.

12. The method according to claim 11, wherein the at least one physical element is a plurality of ribs or a heart.

13. The method according to claim 11, wherein removing or masking out the at least one physical element is based on the disease or the uncertainty in the identification of the disease in the two-dimensional image.

14. The method according to claim 1, wherein identifying the disease includes a use of a trained disease classification model.

15. A system for processing medical computed tomography (CT) data, comprising:
a memory that stores a plurality of instructions; and
a processor that couples to the memory and is configured to execute the plurality of instructions to:
retrieve projection data acquired by scanning a subject;
process the projection data to reconstruct a three-dimensional image;
generate a two-dimensional image based on the three-dimensional image;
identify a disease or an uncertainty in an identification of the disease in the two-dimensional image;
reprocess the projection data or the three-dimensional image into an updated three-dimensional image, wherein at least one parameter of the reprocessing is based on the disease or the uncertainty in the identification of the disease; and
generate an updated two-dimensional image based on the updated three-dimensional image.

## Patentansprüche

1. Computerimplementiertes Verfahren zur Verarbeitung medizinischer Computertomographie- (CT-) Daten, umfassend:
Gewinnen von Projektionsdaten, die durch Scannen eines Probanden erfasst werden;
Verarbeiten der Projektionsdaten, um ein dreidimensionales Bild zu rekonstruieren;
Erzeugen eines zweidimensionalen Bildes basierend auf dem dreidimensionalen Bild;
Identifizieren einer Krankheit oder einer Unsicherheit bei einer Identifizierung der Krankheit im zweidimensionalen Bild;
erneutes Verarbeiten der Projektionsdaten oder des dreidimensionalen Bildes zu einem aktualisierten dreidimensionalen Bild, wobei mindestens ein Parameter der erneuten Verarbeitung auf der Krankheit oder der Unsicherheit bei der Identifizierung der Krankheit basiert; und
Erzeugen eines aktualisierten zweidimensionalen Bildes basierend auf dem aktualisierten dreidimensionalen Bild.

2. Verfahren nach Anspruch 1, wobei das Erzeugen des zweidimensionalen Bildes durch ein neuronales Netzwerk erfolgt.

3. Verfahren nach Anspruch 1, wobei das erneute Verarbeiten der Projektionsdaten oder des dreidimensionalen Bildes durch ein neuronales Netzwerk erfolgt.

4. Verfahren nach Anspruch 1, weiter umfassend das Entrauschen des dreidimensionalen Bildes unter Verwendung eines ersten Wertes für den mindestens einen Parameter.

5. Verfahren nach Anspruch 4, weiter umfassend das Entrauschen des aktualisierten dreidimensionalen Bildes unter Verwendung eines zweiten Wertes für den mindestens einen Parameter, wobei der zweite Wert auf der Krankheit oder der Unsicherheit bei der Identifizierung der Krankheit basiert.

6. Verfahren nach Anspruch 1, weiter umfassend das Anwenden einer auf künstlicher Intelligenz (AI) basierenden Superauflösung auf das dreidimensionale Bild unter Verwendung eines ersten Wertes für den mindestens einen Parameter, sodass das dreidimensionale Bild zu einem hochauflösenden Bild führt.

7. Verfahren nach Anspruch 6, weiter umfassend das Anwenden der Al-basierten Superauflösung auf das aktualisierte dreidimensionale Bild unter Verwendung eines zweiten Wertes für den mindestens einen Parameter, sodass das aktualisierte dreidimensionale Bild zu einem weiteren hochauflösenden Bild führt, wobei der zweite Wert auf der Krankheit oder der Unsicherheit bei der Identifizierung der Krankheit basiert.

8. Verfahren nach Anspruch 1, weiter umfassend das Entrauschen des dreidimensionalen Bildes und/oder des aktualisierten dreidimensionalen Bildes mithilfe eines trainierten konvolutionellen neuronalen Netzwerks (CNN).

9. Verfahren nach Anspruch 1, wobei das Erzeugen des zweidimensionalen Bildes die Verwendung eines digital rekonstruierten Röntgenbild- (DRR-) Netzwerks unter Verwendung eines ersten Wertes für den mindestens einen Parameter umfasst.

10. Verfahren nach Anspruch 9, wobei das Erzeugen des aktualisierten zweidimensionalen Bildes die Verwendung des DRR-Netzwerks unter Verwendung eines zweiten Wertes für den mindestens einen Parameter umfasst, wobei der zweite Wert auf der Krankheit oder der Unsicherheit bei der Identifizierung der Krankheit basiert.

11. Verfahren nach Anspruch 1, weiter umfassend das Identifizieren mindestens eines physikalischen Elements in dem dreidimensionalen Bild und/oder dem aktualisierten dreidimensionalen Bild und das Entfernen oder Ausblenden des mindestens eines physikalischen Elements vor der Erzeugung eines Ausgabebildes.

12. Verfahren nach Anspruch 11, wobei es sich bei dem mindestens einen physikalischen Element um eine Vielzahl von Rippen oder ein Herz handelt.

13. Verfahren nach Anspruch 11, wobei das Entfernen oder Ausblenden des mindestens einen physikalischen Elements auf der Krankheit oder der Unsicherheit bei der Identifizierung der Krankheit im zweidimensionalen Bild basiert.

14. Verfahren nach Anspruch 1, wobei das Identifizieren der Krankheit die Verwendung eines trainierten Krankheitsklassifizierungsmodells einschließt.

15. System zur Verarbeitung medizinischer Computertomographie- (CT-) Daten, umfassend:
einen Speicher, der eine Vielzahl von Anweisungen speichert; und
einen Prozessor, der an den Speicher gekoppelt ist und so konfiguriert ist, dass er die Vielzahl von Anweisungen ausführt zum:
Gewinnen von Projektionsdaten, die durch Scannen eines Probanden erfasst werden;
Verarbeiten der Projektionsdaten, um ein dreidimensionales Bild zu rekonstruieren;
Erzeugen eines zweidimensionalen Bildes basierend auf dem dreidimensionalen Bild;
Identifizieren einer Krankheit oder einer Unsicherheit bei einer Identifizierung der Krankheit im zweidimensionalen Bild;
erneutem Verarbeiten der Projektionsdaten oder des dreidimensionalen Bildes zu einem aktualisierten dreidimensionalen Bild, wobei mindestens ein Parameter der erneuten Verarbeitung auf der Krankheit oder der Unsicherheit bei der Identifizierung der Krankheit basiert; und
Erzeugen eines aktualisierten zweidimensionalen Bildes basierend auf dem aktualisierten dreidimensionalen Bild.

## Revendications

1. Procédé mis en œuvre par ordinateur de traitement de données de tomodensitométrie (TDM) médicale, comprenant :
la récupération de données de projection acquises par numérisation d'un sujet ;
le traitement des données de projection pour reconstruire une image tridimensionnelle ;
la génération d'une image bidimensionnelle sur la base de l'image tridimensionnelle ;
l'identification d'une maladie ou d'une incertitude dans l'identification de la maladie sur l'image bidimensionnelle ;
le retraitement des données de projection ou de l'image tridimensionnelle en une image tridimensionnelle mise à jour, dans lequel au moins un paramètre du retraitement est basé sur la maladie ou sur l'incertitude dans l'identification de la maladie ; et
la génération d'une image bidimensionnelle mise à jour sur la base de l'image tridimensionnelle mise à jour.

2. Procédé selon la revendication 1, dans lequel la génération de l'image bidimensionnelle est effectuée par un réseau neuronal.

3. Procédé selon la revendication 1, dans lequel le retraitement des données de projection ou de l'image tridimensionnelle est effectué par un réseau neuronal.

4. Procédé selon la revendication 1, comprenant en outre le débruitage de l'image tridimensionnelle à l'aide d'une première valeur pour le au moins un paramètre.

5. Procédé selon la revendication 4, comprenant en outre le débruitage de l'image tridimensionnelle mise à jour à l'aide d'une seconde valeur pour le au moins un paramètre, la seconde valeur étant basée sur la maladie ou sur l'incertitude dans l'identification de la maladie.

6. Procédé selon la revendication 1, comprenant en outre l'application, à l'image tridimensionnelle, d'une super-résolution basée sur l'intelligence artificielle (IA) à l'aide d'une première valeur pour le au moins un paramètre de sorte que l'image tridimensionnelle donne lieu à une image haute résolution.

7. Procédé selon la revendication 6, comprenant en outre l'application, à l'image tridimensionnelle mise à jour, de la super-résolution basée sur l'IA à l'aide d'une seconde valeur pour le au moins un paramètre de sorte que l'image tridimensionnelle mise à jour donne lieu à une autre image haute résolution, dans lequel la seconde valeur est basée sur la maladie ou sur l'incertitude dans l'identification de la maladie.

8. Procédé selon la revendication 1 comprend en outre le débruitage de l'image tridimensionnelle et/ou de l'image tridimensionnelle mise à jour par un réseau neuronal convolutif (CNN) entraîné.

9. Procédé selon la revendication 1, dans lequel la génération de l'image bidimensionnelle comprend l'utilisation d'un réseau de radiographies reconstruites numériquement (DRR) à l'aide d'une première valeur pour le au moins un paramètre.

10. Procédé selon la revendication 9, dans lequel la génération de l'image bidimensionnelle mise à jour comprend l'utilisation du réseau DRR à l'aide d'une seconde valeur pour le au moins un paramètre, la seconde valeur étant basée sur la maladie ou sur l'incertitude dans l'identification de la maladie.

11. Procédé selon la revendication 1, comprenant en outre l'identification d'au moins un élément physique dans l'image tridimensionnelle et/ou dans l'image tridimensionnelle mise à jour, et la suppression ou le masquage du au moins un élément physique avant la génération d'une image de sortie.

12. Procédé selon la revendication 11, dans lequel le au moins un élément physique est une pluralité de côtes ou un cœur.

13. Procédé selon la revendication 11, dans lequel la suppression ou le masquage du au moins un élément physique est basé sur la maladie ou sur l'incertitude dans l'identification de la maladie dans l'image bidimensionnelle.

14. Procédé selon la revendication 1, dans lequel l'identification de la maladie inclut l'utilisation d'un modèle de classification de maladie entraîné.

15. Système de traitement de données de tomodensitométrie (TDM) médicale, comprenant :
une mémoire qui stocke une pluralité d'instructions ; et
un processeur qui se couple à la mémoire et est configuré pour exécuter la pluralité d'instructions pour :
récupérer des données de projection acquises par numérisation d'un sujet ;
traiter les données de projection pour reconstruire une image tridimensionnelle ;
générer une image bidimensionnelle sur la base de l'image tridimensionnelle ;
identifier une maladie ou une incertitude dans l'identification de la maladie sur l'image bidimensionnelle ;
retraiter les données de projection ou l'image tridimensionnelle en une image tridimensionnelle mise à jour, dans lequel au moins un paramètre du retraitement est basé sur la maladie ou sur l'incertitude dans l'identification de la maladie ; et
générer une image bidimensionnelle mise à jour sur la base de l'image tridimensionnelle mise à jour.
